# EUROPÄISCHE PATENTSCHRIFT

(11) **EP 0 345 616 B1**
(45) Veröffentlichungstag und Bekanntmachung des Hinweises auf die Patenterteilung: **15.11.1995**
(21) Anmeldenummer: 89109772.7
(22) Anmeldetag: 30.05.1989
(51) Int. Cl.: C07K 17/10, A61K 38/55, A61K 47/00

(54) **Hirudin-Derivate mit verzögerter Wirkung**
Hirudin derivatives with delayed action
Dérivés de l'hirudine à effet retard

(30) Priorität: 04.06.1988 DE 3819079
(43) Veröffentlichungstag der Anmeldung: 13.12.1989
(73) Patentinhaber: HOECHST AKTIENGESELLSCHAFT, 65926 Frankfurt am Main (DE)
(72) Erfinder: Crause, Peter, Dr., D-6050 Offenbach (DE); Habermann, Paul, Dr., D-6239 Eppstein/Taunus (DE); Kramer, Martin, Prof.-Dr., D-6200 Wiesbaden (DE); Obermeier, Rainer, Dr., D-6234 Hattersheim am Main (DE); Sauber, Klaus, Dr., D-6232 Bad Sodenn am Taunus (DE); Tripier, Dominique, Dr., D-6239 Eppstein/Taunus (DE)

(56) Entgegenhaltungen:
- EP-A- 0 158 986
- EP-A- 0 187 361
- EP-A- 0 209 061

## Beschreibung

Die Erfindung betrifft Hirudin-Derivate, die eine verzögerte Wirkung zeigen, Verfahren zu deren Herstellung sowie deren Verwendung.

Hirudine sind z.B. bekannt aus EP-A 142 860, EP-A- 158 564, EP-A 158 986, EP-A 168 342, EP-A 171 024, EP-A 193 175, EP-A 200 655, EP-A 209 061, EP-A 227 938, DE 34 45 517 A1, DE 38 05 540.6 und Chang, FEBS, Bd. 164 (1983) 307. Chang zeigte u.a., daß C-terminale Verkürzungen die antithrombotische Wirkung des Hirudins stark beeinträchtigen. Auch die Bedeutung von Hirudinen für die Antikoaglationstherapie wurde hinlänglich beschrieben (z.B. P. Walsmann und F. Markwardt; Pharmazie, 36 (1981) 653). So inhibiert es spezifisch Thrombin, ist aber ansonsten pharmakologisch inert, d.h. unerwünschte Nebenwirkungen wurden bisher nicht festgestellt. Als nachteilig für die medizinische Verwendung als Thromboseprophylaktikum kann jedoch die relativ kurze Verweildauer der Hirudine im tierischen oder menschlichen Körper (Richter et al., Haematol. 115 (1988) 64; Markwardt et al., Pharmazie 43 (1988) 202) angesehen werden. EP-A 0 187 361 beschreibt nun Präparate mit verzögerter Wirkung durch Zusatz eines Depotträgers. Veronese et al. (Applied Biochemistry and Biotechnology Vol. 11, 141 bis 152, 1985) beschreiben zudem die PEG-Derivatisierung der Enzyme Ribonuclease und Superoxid-Dismutase.

Der Erfindung lag daher die Aufgabe zugrunde, neue Hirudin-Derivate zu finden, die eine längere Halbwertszeit aufweisen, oder deren Elimination gering ist. Diese Aufgabe wird überraschenderweise durch Hirudin-Derivate, gebildet aus Hirudin oder dessen physiologisch annehmbarem Salz und einem Träger, gelöst. Als Hirudine kommen beispielsweise die in den auf Seite 1 zitierten Literaturstellen beschriebenen Verbindungen in Betracht, insbesondere die in EP-A 171 024, EP-A 158 986, EP-A 209 061 und DE 38 05 540.6 beschriebenen Verbindungen, wie z.B. 
0    1                                                    10
Leu-Thr-Tyr-Thr-Asp-Cys-Thr-Glu-Ser-Gly-Gln-Asn-Leu-Cys-
                                20
Leu-Cys-Glu-Gly-Ser-Asn-Val-Cys-Gly-Gln-Gly-Asn-Lys-Cys-
            30                                          40
Ile-Leu-Gly-Ser-Asp-Gly-Glu-Lys-Asn-Gln-Cys-Val-Thr-Gly-
                                          50
Glu-Gly-Thr-Pro-Lys-Pro-Gln-Ser-His-Asn-Asp-Gly-Asp-Phe-
                      60
Glu-Glu-Ile-Pro-Glu-Glu-Tyr-Leu-Gln.
Diese Hirudine können nach dem Fachmann allgemein bekannten Methoden der Peptidchemie, siehe z.B. Houben-Weyl, Methoden der organischen Chemie, Band 15/2, bevorzugt mittels Festphasensynthese wie z.B. von B. Merifield, J.Am.Chem.Soc. 85, 2149 (1963) oder R.C. Sheppard, Int. J. Peptide Protein Res. 21, 118 (1983) beschrieben oder durch äquivalente bekannte Methoden hergestellt werden. Alternativ sind die genannten Hirudine auch durch dem Fachmann bekannte gentechnische Methoden zugänglich.

Weiterhin kommen folgende mittels gentechnologischer Methoden veränderte Hirudine in Betracht. Vorteilhaft solche, wobei z.B. zwei der drei natürlich in der Sequenz vorkommenden Lysine durch eine natürliche Aminosäure, bevorzugt Asg, Asn, ersetzt wurden. Ferner, auch solche Hirudine, die durch gezielte N- (bevorzugt Lys) bzw. C-terminale (bevorzugt Met) Verlängerung verändert wurden. Bei einer N-terminalen Verlängerung sind die im Verlauf der Sequenz vorkommenden Lysine durch eine andere natürliche Aminosäure, bevorzugt Asn, Arg, ersetzt.

Als Träger werden lösliche Träger, insbesondere Polysaccharide, wie z.B. Dextrane (MW 20000-75000 dt), -bevorzugt Dextrane (MW 70000 dt)- Laevane, Heparine (MW 6000-20000 dt) oder "low molecular weight" Heparine (MW <6000 dt), Polyethylenglykole (MW 1500-15000 dt) oder Gelatinepartialhydrolysate beispielsweise mit Diisocyanaten vernetzt Gelatinepartialhydrolysate (Polygeline, ®Haemaccel), oder unlösliche Träger, wie z.B. Sepharosen, beispielsweise CH-Sepharose 4B® (Fa. Pharmacia), Agarosen, Cellulose, Hydroxymethacrylate, die alle nach bekannten Verfahren aktiviert wurden, verwendet, insbesondere jedoch Dextrane, Heparine und low molecular weight Heparine.

Das Verhältnis Hirudin zu Träger kann in den Hirudin-Derivaten sehr stark variieren. Es ist abhängig von der Art des Trägers und den Reaktionsbedinungen.

Die Erfindung betrifft auch ein Verfahren zur Herstellung der Hirudin-Derivate, das dadurch gekennzeichnet ist, daß Hirudine einem aktiven Träger bei einer Temperatur von 0 °C bis 25 °C, bevorzugt bei 4 °C, umgesetzt werden.

Die erfindungsgemäßen Hirudin-Derivate sind wertvolle Thrombin-Inhibitoren, die sich insbesondere durch eine verlängerte Halbwertszeit auszeichnen, was wiederum eine umfassende Anwendung z.B. in der Thromboseprophylaxe ermöglicht.

So werden die Hirudine und Hirudin-Derivate beispielsweise vorteilhaft verwendet bei der Oberflächenbeschichtung von Herzklappen, (z.B. aus Kunststoff), künstlichen Gefäßen, Kathedern von biokompatiblen medizinischen Geräten oder von Filtern, Membranen und Materialien wie z.B. Keramiken, der gebräuchlichen Hämdialyseapparaturen.
Bei der Bestimmung und dem Nachweis von relevanten Blutkomponenten stellt sich das Problem, daß das zu untersuchende Blut vorbehandelt werden muß. Dies kann Probleme für die Aussagekraft solcher Tests mit sich bringen. Teströhrchen, die mit Hirudin beschichtet sind, eröffnen dem Praktiker neue Möglichkeiten.

Ferner finden Hirudine und Hirudin-Derivate Anwendung in Implantaten, die u.a. auch für die verzögerte Freisetzung des Wirkstoffs verwendet werden, z.B. osmotische Minipumpen, biologisch abbaubare Mikrokapseln, Rods, Liposomenpräparate.

Die erfindungsgemäßen Hirudin-Derivate zeigen, daß sich Hirudine mit hochmolekularen Trägermaterialien umsetzen lassen, wobei überraschenderweise die Hirudinaktivität erhalten bleibt. Das pharmakokinetische Verhalten des Hirudins ändert sich vorteilhaft. Injiziert man Hirudin und Dextran-Hirudin in einem vergleichenden Experiment Ratten und mißt mittels eines Thrombinhemmtestes die Plasmakonzentration, so sit nach einer Stunde mit der ca. 10-fach geringeren Menge an Dextran-Hirudin eine Thrombinhemmung meßbar. Ein weiterer Vorteil liegt überraschend in der Verlängerung der Halbwertzeit. Sie beträgt für Dextran-Hirudin 6 bis 7 Stunden, während sie für Hirudin 1 bis 2 Stunden (Markwardt et al. Pharmazie 43 (1988) 202) beträgt.

Die nachfolgenden Beispiele, in denen das in der deutschen Patentanmeldung 38 05 5406 beschriebene Isohirudin ('Leu-Thr-Hirudin') verwendet wird, sollen die Erfindung erläutern, jedoch ohne auf diese einzuschränken. Es ist dem Fachmann geläufig, daß alle bekannten Hirudine äquivalent zu dem eingesetzten Hirudin mit einem Träger umgesetzt werden können und auch die auf Seite 2 beschriebenen Hirudine. Dabei können sich die Bedingungen für einzelne Reaktionen ändern.

### Beispiel 1 Herstellung von Hirudin

Die Synthese von Hirudin erfolgt beispielsweise in Hefezellen, die Hirudin gemäß der deutschen Patentanmeldung P 38 05 540.6 (HOE 88/F 043) ausschleusen. Hiurudin wird anschließend über HP20-Säulenchromatographie (deutsche Patentanmeldung P 37 38 541.0; HOE 87/F 337) angereichert. Nach Dialyse und anschließender Affinitätschromatographie über Thrombin-Sepharose® (Walsmann, P.: Pharmazie 36 (1981) 860-861) erfolgt die endgültige Reinigung über "reversed phase" HPLC.

Die Synthese kann aber auch mittels anderer bekannter gentechnischer oder peptidchemischer Methoden erfolgen.

### Beispiel 2 Applikation von Hirudin und anschließender Thrombin-Hemmtest

Weibliche und männliche Wistar-Ratten mit einem Körpergewicht von ca. 300 g werden mit Ethylurethan (1,5 g/kg i.p.) betäubt. Den Ratten werden 1000 AT-U/kg von einem Hirudin-Derivat bzw. authentischem 'Leu-Thr-Hirudin', welches in physiologischer Kochsalzlösung gelöst war, i.v. appliziert. Zur Blutentnahme wird den Tieren ein Katheder in die Karotis implantiert. Zur Bestimmung der Anti-Thrombinaktivität wird den Tieren Blut entnommen und mit Citrat behandelt. 0,1 ml des so behandelten Blutes werden mit 0,2 ml Tris/HCl-Puffer (0,1 mol/l; pH 7,4) gemischt und bei 37°C vorinkubiert. Nach Zusatz von 0,1 ml einer Thrombinlösung (10 NIH-U/ml) wird die Gerinnungszeit mittels eines Koagulometers nach Schnitger und Gross bestimmt. Parallel zu dem Experiment wird eine Eichkurve mit Referenzplasma angelegt, aus der die jeweilige Plasma-Hirudin- bzw.
Plasma-Hirudin-Derivat-Konzentration abgelesen wird.

### Beispiel 3 Dextran-Hirudin

Die 'Kopplung' von 'Leu-Thr-Hirudin' an Dextran (MW 70000 dt) erfolgt nach der Methode von Parikh, J. et al. Meth. Enzymol 34 (1974) 77. Dazu wird das Dextran durch Behandlung mit Meta-Perjodat bei 4°C aktiviert und anschließend dialysiert und lyophilisiert. 2 g aktiviertes Dextran werden dann in 285 ml 0,1 mol/l Natriumphosphat-Puffer (pH 8,8) bei Raumtemperatur gelöst, die Lösung auf 4°C abgekühlt, 20 mg Hirudin zugegeben und 14 Stunden bei 4°C inkubiert. Danach werden 75 ml abgetrennt und lyphilisiert. nachdem die wiedermolekularen Puffersalze durch Gelfiltration (®Sephadex G-25, 1 cm x 100 cm) entfernt wurden, wird die Lösung erneut lyphilisiert.
Die Reaktion findet mindestens zwischen einem der drei Lysine und dem aktiviertem Dextran statt.

Zur Reduktion der gebildeten Schiffschen Base werden 75 mg NaBH₄ zu den 75 ml der inkubierten Lösung gegeben, 40 Minuten bei 4°C gerührt und anschließend die Lösung gegen Wasser für 24 Stunden bei 4°C dialysiert (Memmbran: Servapor Dialysis Lubing; Diameter 10 mm, Fa. Serva Feinbiochemica GmbH Heidelberg). Dann eine Gelfiltration über ®Sephadex G-25 und eine Lyphilisation.
Das Dextran-Hirudin wird mittels Thrombin-Sepharose®-Affinitätschromatographie entsprechend Beispiel 1 gereinigt. Die Bestimmung der Dextran-Hirudin-Aktivität erfolgt gemäß der Methode von Griesbach et al. (Thromb. Res. 37 (1985) 347-350). Dabei wird die Hemmung der Thrombin-katalysierten Spaltung von Chromozym TH® gemessen. Bezogen auf die molare Basis entspricht die gemessene Aktivität der von Hirudin. Das so gereinigte Dextran-Hirudin wird gemäß Beispiel 2 Wistar-Ratten i.v. appliziert. Nach 1 Stunde wird eine entdeckbare Konzentration im Plasma der Ratten gefunden. Dies kann im Vergleichsexperiment nur mit der ca. 10fach höheren Dosis von Hirudin beschrieben werden. Verfolgt man die Hirudinkonzentration des Plasmas weiter, so findet man eine Halbwertszeit der Eliminierung der Substanz von 6-7 Stunden. Dies ist eine deutliche Verlängerung gegenüber Hirudinen (1-3 Stunden).
Die verwendeten Versuchstiere zeigen keine Beeinträchtigung in ihrem Befinden.

### Beispiel 4 Sepharose®-Hirudin

8 mg Hirudin werden in 2 ml 0,1 M NaHCO₃ und 0,5 M NaCl bei pH = 8,0 gelöst. 400 mg aktivierte CH-Sepharose 4 B® (Pharmacia) werden nach Herstellervorschriften gequollen. Dann wird die Proteinlösung zu dem Träger gegeben und 1,5 Stunden bei 23° stehengelassen. Danach wird abgesaugt und das Immobilisat zur Inaktivierung restlicher Bindungsgruppen 1 Stunde lang in 0,1 M TRIS, pH = 8,0, stehengelassen. Anschließend wird der Träger nach Herstellungsvorschrift gewaschen.

Die Protein-Kopplungsausbeute beträgt 5 %. Die biologische Aktivität wird in Nitro nach Beispiel 2 bestimmt. Aus dem behandelten Plasma wird das Sepharose®-Hirudin isoliert, mit einer 1 bis 1,5 M NaCl-Lösung gewaschen und erneut im Hemmtext eingesetzt. Die gemessene Aktivität liegt innerhalb der Fehlergrenze der 1. Messung.

## Patentansprüche (Patentansprüche für folgende(n) Vertragsstaat(en): AT, BE, CH, DE, FR, GB, IT, LI, LU, NL, SE)

1. Hirudin-Derivate, gebildet aus Hirudin oder dessen physiologisch annehmbaren Salz und einem Träger mit Ausnahme von CNBr-aktivierter Sepharose CL-4B und hydroxymethyliertem Polystyrol-Harz, dadurch gekennzeichnet, daß Hirudin an den Träger chemisch gebunden ist.

2. Hirudin-Derivat gemäß Anspruch 1, dadurch gekennzeichnet, daß der Träger ein Polysaccharid ist.

3. Hirudin-Derivat gemäß Anspruch 1 oder 2, dadurch gekennzeichnet, daß der Träger ein Dextran oder ein Heparin ist.

4. Hirudin-Derivat gemäß einem oder mehreren der Ansprüche 1 bis 3, dadurch gekennzeichnet, daß der Träger "low molecular weight" Heparin ist.

5. Hirudin-Derivat gemäß Anspruch 1, dadurch gekennzeichnet, daß der Träger ein Polyethylenglykol (MW 1500 bis 15000 dt) oder ein Gelatinepartialhydrolysat ist.

6. Hirudin-Derivat gemäß einem oder mehreren der Ansprüche 1 bis 4, dadurch gekennzeichnet, daß das Hirudin die Sequenz

0 1 10
Leu-Thr-Tyr-Thr-Asp-Cys-Thr-Glu-Ser-Gly-Gln-Asn-Leu-Cys-
20
Leu-Cys-Glu-Gly-Ser-Asn-Val-Cys-Gly-Gln-Gly-Asn-Lys-Cys-
30 40
Ile-Leu-Gly-Ser-Asp-Gly-Glu-Lys-Asn-Gln-Cys-Val-Thr-Gly-
50
Glu-Gly-Thr-Pro-Lys-Pro-Gln-Ser-His-Asn-Asp-Gly-Asp-Phe-

60
Glu-Glu-Ile-Pro-Glu-Glu-Tyr-Leu-Gln besitzt.

7. Hirudin-Derivat gemäß Anspruch 6, dadurch gekennzeichnet, daß in dem Hirudin Lys 26 und Lys 35 oder Lys 35 und Lys 46 oder Lys 26 und Lys 46 jeweils durch eine natürliche Aminosäure ersetzt sind.

8. Hirudin-Derivat gemäß Anspruch 6 oder 7, dadurch gekennzeichnet, daß in dem Hirudin Lys 26 durch Asn und Lys 35 oder Lys 46 durch Arg ersetzt sind.

9. Hirudin-Derivat gemäß Anspruch 6, dadurch gekennzeichnet, daß das Hirudin N-terminal nun verlängert ist und Lys 26, Lys 35 und Lys 46 durch andere natürliche Aminosäuren ersetzt sind.

10. Hirudin-Derivat gemäß einem oder mehreren der Ansprüche 1 bis 9, dadurch gekennzeichnet, daß das Hirudin C-terminal um eine natürliche Aminosäure verlängert ist.

11. Verfahren zur Herstellung eines Hirudin-Derivates gemäß einem oder mehreren der Ansprüche 1 bis 10, dadurch gekennzeichnet, daß Hirudin mit einem aktivierten Träger bei einer Temperatur von 0°C bis 25°C umgesetzt wird.

12. Verwendung von Hirudin-Derivaten, gebildet aus Hirudin oder dessen physiologisch annehmbaren Salz und einem Träger, wobei Hirudin an dem Träger chemisch gebunden ist, als Thrombin-Inhibitor.

## Patentansprüche (Patentansprüche für folgende(n) Vertragsstaat(en): ES, GR)

1. Verfahren zur Herstellung eines Hirudin-Derivats, gebildet aus Hirudin oder dessen physiologisch annehmbaren Salz und einem Träger mit Ausnahme von CNBr-aktivierter Sepharose CL-4B und hydroxymethyliertem Polystyrol-Harz, dadurch gekennzeichnet, daß Hirudin mit einem aktivierten Träger bei einer Temperatur von 0°C bis 25°C umgesetzt wird, wobei Hirudin an den Träger chemisch gebunden wird.

2. Verfahren gemäß Anspruch 1, dadurch gekennzeichnet, daß der Träger ein Polysaccharid ist.

3. Verfahren gemäß Anspruch 1 oder 2, dadurch gekennzeichnet, daß der Träger ein Dextran oder ein Heparin ist.

4. Verfahren gemäß einem oder mehreren der Ansprüche 1 bis 3, dadurch gekennzeichnet, daß, der Träger "low molecular weight" Heparin ist.

5. Verfahren gemäß Anspruch 1, dadurch gekennzeichnet, daß der Träger ein Polyethylenglykol (MW 1500 bis 15000 dt) oder ein Gelatinepartialhydrolysat ist.

6. Verfahren gemäß einem oder mehren der Ansprüche 1 bis 4, dadurch gekennzeichnet, daß das Hirudin die Sequenz

0     1                                                    10
Leu-Thr-Tyr-Thr-Asp-Cys-Thr-Glu-Ser-Gly-Gln-Asn-Leu-Cys-
                                        20
Leu-Cys-Glu-Gly-Ser-Asn-Val-Cys-Gly-Gln-Gly-Asn-Lys-Cys-
          30                                                    40
Ile-Leu-Gly-Ser-Asp-Gly-Glu-Lys-Asn-Gln-Cys-Val-Thr-Gly-
                                                  50
Glu-Gly-Thr-Pro-Lys-Pro-Gln-Ser-His-Asn-Asp-Gly-Asp-Phe-

60
Glu-Glu-Ile-Pro-Glu-Glu-Tyr-Leu-Gln besitzt.

7. Verfahren gemäß Anspruch 6, dadurch gekennzeichnet, daß in dem Hirudin Lys 26 und Lys 35 oder Lys 35 und Lys 46 oder Lys 26 und Lys 46 jeweils durch eine natürliche Aminosäure ersetzt sind.

8. Verfahren gemäß Anspruch 6 oder 7, dadurch gekennzeichnet, daß in dem Hirudin Lys 26 durch Asn und Lys 35 oder Lys 46 durch Arg ersetzt sind.

9. Verfahren gemäß Anspruch 6, dadurch gekennzeichnet, daß das Hirudin N-terminal um eine natürliche Aminosäure verlängert ist und Lys 26, Lys 35 und Lys 46 durch eine andere natürliche Aminosäure ersetzt sind.

10. Verfahren gemäß einem oder mehreren der Ansprüche 1 bis 9, dadurch gekennzeichnet, daß das Hirudin C-terminal um eine natürliche Aminosäure verlängert ist.

11. Verwendung von Hirudin-Derivaten, gebildet aus Hirudin oder dessen physiologisch annehmbaren Salz und einem Träger, wobei Hirudin an dem Träger chemisch gebunden ist, als Thrombin-Inhibitor.

## Claims (Claims for the following Contracting State(s): AT, BE, CH, DE, FR, GB, IT, LI, LU, NL, SE)

1. A hirudin derivative formed of hirudin or the physiologically acceptable salt thereof and a carrier with the exception of CNBr-activated Sepharose CL-4B and hydroxymethylated polystyrene resin, wherein hirudin is chemically linked to the carrier.

2. A hirudin derivative as claimed in claim 1, wherein the carrier is a polysaccharide.

3. A hirudin derivative as claimed in claim 1 or 2, wherein the carrier is a dextran or a heparin.

4. A hirudin derivative as claimed in one or more of claims 1 to 3, wherein the carrier is low molecular weight heparin.

5. A hirudin derivative as claimed in claim 1, wherein the carrier is a polyethylene glycol (MW 1500 to 15,000 dt) or a gelatin partial hydrolysate.

6. A hirudin derivative as claimed in one or more of claims 1 to 4, wherein the hirudin has the sequence

0 1 10
Leu-Thr-Tyr-Thr-Asp-Cys-Thr-Glu-Ser-Gly-Gln-Asn-Leu-Cys-
20
Leu-Cys-Glu-Gly-Ser-Asn-Val-Cys-Gly-Gln-Gly-Asn-Lys-Cys-

30                                              40
Ile-Leu-Gly-Ser-Asp-Gly-Glu-Lys-Asn-Gln-Cys-Val-Thr-Gly-
                                    50
Glu-Gly-Thr-Pro-Lys-Pro-Gln-Ser-His-Asn-Asp-Gly-Asp-Phe-
                60
Glu-Glu-Ile-Pro-Glu-Glu-Tyr-Leu-Gln.

7. A hirudin derivative as claimed in claim 6, wherein Lys 26 and Lys 35 or Lys 35 and Lys 46 or Lys 26 and Lys 46 in the hirudin are in each case replaced by a natural amino acid.

8. A hirudin derivative as claimed in claim 6 or 7, wherein there is replacement in the hirudin of Lys 26 by Asn and Lys 35 or Lys 46 by Arg.

9. A hirudin derivative as claimed in claim 6, wherein the hirudin now has an N-terminal extension, and Lys 26, Lys 35 and Lys 46 are replaced by other natural amino acids.

10. A hirudin derivative as claimed in one or more of claims 1 to 9, wherein the hirudin has a C-terminal extension by one natural amino acid.

11. A process for the preparation of a hirudin derivative as claimed in one or more of claims 1 to 10, which comprises hirudin being reacted with an activated carrier at a temperature of 0°C to 25°C.

12. The use of hirudin derivatives formed of hirudin or the physiologically acceptable salt thereof and a carrier, hirudin being chemically linked to the carrier, as thrombin inhibitor.

## Claims (Claims for the following Contracting State(s): ES, GR)

1. A process for the preparation of a hirudin derivative formed of hirudin or the physiologically acceptable salt thereof and a carrier with the exception of CNBr-activated Sepharose CL-4B and hydroxymethylated polystyrene resin, which comprises hirudin being reacted with an activated carrier at a temperature of 0°C to 25°C, hirudin being chemically linked to the carrier.

2. The process as claimed in claim 1, wherein the carrier is a polysaccharide.

3. The process as claimed in claim 1 or 2, wherein the carrier is a dextran or a heparin.

4. The process as claimed in one or more of claims 1 to 3, wherein the carrier is low molecular weight heparin.

5. The process as claimed in claim 1, wherein the carrier is a polyethylene glycol (MW 1500 to 15,000 dt) or a gelatin partial hydrolysate.

6. The process as claimed in one or more of claims 1 to 4, wherein the hirudin has the sequence

0 1 10
Leu-Thr-Tyr-Thr-Asp-Cys-Thr-Glu-Ser-Gly-Gln-Asn-Leu-Cys-
20
Leu-Cys-Glu-Gly-Ser-Asn-Val-Cys-Gly-Gln-Gly-Asn-Lys-Cys-

30                                                    40
Ile-Leu-Gly-Ser-Asp-Gly-Glu-Lys-Asn-Gln-Cys-Val-Thr-Gly-
                                        50
Glu-Gly-Thr-Pro-Lys-Pro-Gln-Ser-His-Asn-Asp-Gly-Asp-Phe-
            60
Glu-Glu-Ile-Pro-Glu-Glu-Tyr-Leu-Gln.

7. The process as claimed in claim 6, wherein Lys 26 and Lys 35 or Lys 35 and Lys 46 or Lys 26 and Lys 46 in the hirudin are in each case replaced by a natural amino acid.

8. The process as claimed in claim 6 or 7, wherein there is replacement in the hirudin of Lys 26 by Asn and Lys 35 or Lys 46 by Arg.

9. The process as claimed in claim 6, wherein the hirudin has an N-terminal extension by one natural amino acid, and Lys 26, Lys 35 and Lys 46 are replaced by another natural amino acid.

10. The process as claimed in one or more of claims 1 to 9, wherein the hirudin has a C-terminal extension by one natural amino acid.

11. The use of hirudin derivatives formed of hirudin or the physiologically acceptable salt thereof and a carrier, hirudin being chemically linked to the carrier, as thrombin inhibitor.

## Revendications (Revendications pour l'(les) Etat(s) contractant(s) suivant(s): AT, BE, CH, DE, FR, GB, IT, LI, LU, NL, SE)

1. Dérivés d'hirudine, constitués d'hirudine ou d'un de ses sels physiologiquement acceptables, et d'un support, à l'exception du Sepharose CL-4B activé au CNBr et d'une résine polystyrène hydroxyméthylée, caractérisés en ce que l'hirudine est liée chimiquement au support.

2. Dérivé d'hirudine selon la revendication 1, caractérisé en ce que le support est un polysaccharide.

3. Dérivé d'hirudine selon la revendication 1 ou 2, caractérisé en ce que le support est un dextran ou une héparine.

4. Dérivé d'hirudine selon une ou plusieurs des revendications 1 à 3, caractérisé en ce que le support est une héparine "à faible masse moléculaire".

5. Dérivé d'hirudine selon la revendication 1, caractérisé en ce que le support est un polyéthylèneglycol (PM: 1 500 à 15 000 Da) ou un hydrolysat partiel de gélatine.

6. Dérivé d'hirudine selon une ou plusieurs des revendications 1 à 4, caractérisé en ce que l'hirudine comporte la séquence

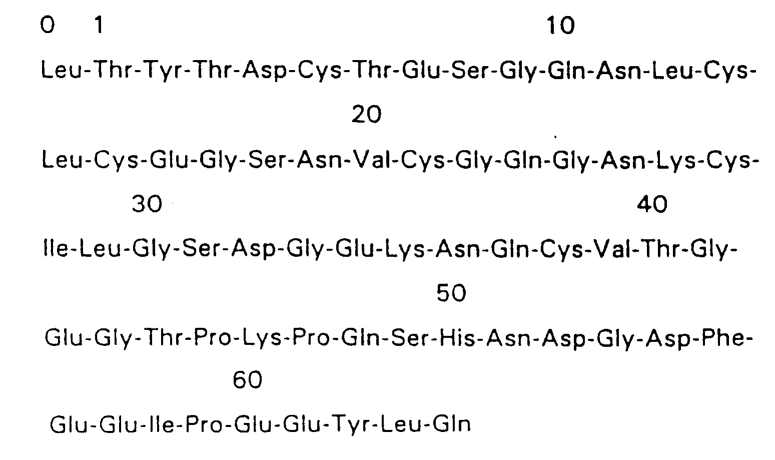

7. Dérivé d'hirudine selon la revendication 6, caractérisé en ce que, dans l'hirudine, les restes Lys 26 et Lys 35 ou Lys 35 et Lys 46 ou Lys 26 et Lys 46 sont remplacés chacun par un aminoacide naturel.

8. Dérivé d'hirudine selon la revendication 6 ou 7, caractérisé en ce que, dans l'hirudine, le reste Lys 26 est remplacé par Asn et le reste Lys 35 ou Lys 46 est remplacé par Arg.

9. Dérivé d'hirudine selon la revendication 6, caractérisé en ce que l'hirudine est prolongée à l'extrémité N-terminale, et les restes Lys 26, Lys 35 et Lys 46 sont remplacés par d'autres aminoacides naturels.

10. Dérivé d'hirudine selon une ou plusieurs des revendications 1 à 9, caractérisé en ce que l'hirudine est prolongée à l'extrémité C-terminale par un aminoacide naturel.

11. Procédé pour la préparation d'un dérivé d'hirudine selon une ou plusieurs des revendications 1 à 10, caractérisé en ce que l'on fait réagir l'hirudine avec un support activé, à une température de 0 à 25°C.

12. Utilisation de dérivés d'hirudine constitués d'hirudine ou d'un de ses sels physiologiquement acceptables et d'un support, l'hirudine étant liée chimiquement au support, en tant qu'inhibiteur de la thrombine.

## Revendications (Revendications pour l'(les) Etat(s) contractant(s) suivant(s): ES, GR)

1. Procédé pour la préparation d'un dérivé d'hirudine constitué d'hirudine ou d'un de ses sels physiologiquement acceptables et d'un support, à l'exception du Sepharose CL-4B activé au CNBr et d'une résine polystyrène hydroxyméthylée, caractérisé en ce que l'on fait réagir l'hirudine avec un support activé, à une température de 0 à 25°C, ce par quoi l'hirudine est liée chimiquement au support.

2. Procédé selon la revendication 1, caractérisé en ce que le support est un polysaccharide.

3. procédé selon la revendication 1 ou 2, caractérisé en ce que le support est un dextran ou une héparine.

4. Procédé selon une ou plusieurs des revendications 1 à 3, caractérisé en ce que le support est une héparine "à faible masse moléculaire".

5. Procédé selon la revendication 1, caractérisés en ce que le support est un polyéthylèneglycol (PM: 1 500 à 15 000 Da) ou un hydrolysat partiel de gélatine.

6. Procédé selon une ou plusieurs des revendications 1 à 4, caractérisé en ce que l'hirudine comporte la séquence

0 1 10
Leu-Thr-Tyr-Thr-Asp-Cys-Thr-Glu-Ser-Gly-Gln-Asn-Leu-Cys-
20
Leu-Cys-Glu-Gly-Ser-Asn-Val-Cys-Gly-Gln-Gly-Asn-Lys-Cys-
30 40
Ile-Leu-Gly-Ser-Asp-Gly-Glu-Lys-Asn-Gln-Cys-Val-Thr-Gly-
50
Glu-Gly-Thr-Pro-Lys-Pro-Gln-Ser-His-,Asn-Asp-Gly-Asp-Phe-
60
Glu-Glu-Ile-Pro-Glu-Glu-Tyr-Leu-Gln

7. Procédé selon la revendication 6, caractérisé en ce que, dans l'hirudine, les restes Lys 26 et Lys 35 ou Lys 35 et Lys 46 ou Lys 26 et Lys 46 sont remplacés chacun par un aminoacide naturel.

8. Procédé selon la revendication 6 ou 7, caractérisé en ce que, dans l'hirudine, le reste Lys 26 est remplacé par Asn et le reste Lys 35 ou Lys 46 est remplacé par Arg.

9. Procédé selon la revendication 6, caractérisé en ce que l'hirudine est prolongée à l'extrémité N-terminale par un aminoacide naturel, et les restes Lys 26, Lys 35 et Lys 46 sont remplacés par d'autres aminoacides naturels.

10. Procédé selon une ou plusieurs des revendications 1 à 9, caractérisé en ce que l'hirudine est prolongée à l'extrémité C-terminale par un aminoacide naturel.

11. Utilisation de dérivés d'hirudine constitués d'hirudine ou d'un de ses sels physiologiquement acceptables et d'un support, l'hirudine étant liée chimiquement au support, en tant qu'inhibiteur de la thrombine.
